# EUROPEAN PATENT APPLICATION

(11) **EP 4 803 512 A1**
(43) Date of publication of application: **09.09.2026**
(21) Application number: 24884926.7
(22) Date of filing: 31.10.2024
(51) Int. Cl.: C07D 401/04, A61K 31/444, A61P 25/00

(54) **COMPOUND CAPABLE OF INHIBITING CONNEXIN-ASSOCIATED KINASE 1, PREPARATION, AND USE THEREOF**

(30) Priority: 02.11.2023 CN 202311445800
(71) Applicant: Xizang Haisco Pharmaceutical Co., Ltd., Shannan, Xizang 856099 (CN)
(72) Inventor: LI, Yao, Lhoka, Tibet 856099 (CN); SHI, Zongjun, Lhoka, Tibet 856099 (CN); REN, Lei, Lhoka, Tibet 856099 (CN); CHENG, Fengkai, Lhoka, Tibet 856099 (CN); TANG, Pingming, Lhoka, Tibet 856099 (CN); ZHANG, Chen, Lhoka, Tibet 856099 (CN); YAN, Pangke, Lhoka, Tibet 856099 (CN)
(74) Representative: AWA Denmark A/S
(86) International application number: PCT/CN2024/129056
(87) International publication number: WO 2025/092928

(57) **Abstract**

The present invention relates to an AAK1 inhibitor, a preparation method therefor, and a use thereof. In particular, the present invention relates to a compound represented by general formula (I), a preparation method therefor, a pharmaceutical composition thereof, and a use of the compound represented by general formula (I) or a pharmaceutical composition thereof in the preparation of a medicament for preventing and/or treating AAK1-mediated diseases, wherein substituents in general formula (I) have the same definitions as in the description.

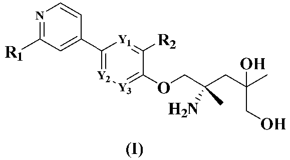

## Description

The present invention claims the right of priority for the earlier application with the patent application number of CN 202311445800.X, entitled "Compound Capable of Inhibiting Connexin-Associated Kinase 1, Preparation, and Use Thereof" and submitted to the China National Intellectual Property Administration on 2 November 2023.

### Technical Field

The present invention belongs to the field of pharmaceuticals, and particularly relates to a compound capable of inhibiting adaptor associated kinase 1 (AAK1), a preparation method therefor, and a composition and the use thereof in the preparation of a medicament.

### Background Art

Adaptor associated kinase 1 (AAK1) is a member of the Ark1/Prk1 family of serine/threonine kinases. AAK1 mRNA exists in two splice forms known as the short form and the long form. The long form is predominant and is highly expressed in the brain and heart. AAK1 is enriched in the synaptosomal preparation and is co-localised with endocytic structures in cultured cells. AAK1 regulates the endocytosis involving clathrin coats, a key process in synaptic vesicle recycling and receptor-mediated endocytosis. AAK1 binds to an AP2 complex, which is a heterotetramer connecting the receptor cargo with the clathrin coat. Clathrin-AAK1 binding stimulates AAK1 activity. AAK1 phosphorylates the mu-2 subunit of AP-2, which enhances the binding of mu-2 to tyrosine-containing sorting motifs on the cargo receptor. Phosphorylation of Mu2 is not required for receptor uptake, but improves the internalisation efficiency.

Studies have shown that AAK1 knockout mice exhibit a significantly reduced response to persistent pain in phase II of the formalin model, and significantly attenuate mechanical allodynia induced by spinal nerve ligation (SNL). The AAK1 small-molecule inhibitor LP-935509 can significantly attenuate pain responses in phase II of the formalin model in mice, mechanical allodynia induced by SNL in mice, as well as pain responses in the mouse chronic constriction injury (CCI) model and the mouse streptozotocin-induced diabetic neuropathy model (Kostich et al., J Pharmacol Exp Ther, 2016). These study results show that the inhibition of AAK1 activity may have a potential therapeutic effect on pain.

AAK1 has been identified as an inhibitor of neuregulin-1/ErbB4 signalling in PC12 cells. Loss of AAK1 expression via RNA interference-mediated gene silencing or by treatment with the kinase inhibitor K252a (which inhibits AAK1 activity) potentiates neuregulin-1-induced neurite outgrowth. Such treatments result in increased ErbB4 expression and increased ErbB4 accumulation in the plasma membrane or in close proximity to the plasma membrane. NRG1 and ErbB4 are putative susceptibility genes of schizophrenia. The SNPs in both genes are associated with multiple endophenotypes of schizophrenia. Mouse models for neuregulin-1 and ErbB4KO have displayed morphological changes and behavioural manifestations related to schizophrenia. Furthermore, the single nucleotide polymorphism in the introns of the AAK1 gene has been implicated with the onset age in Parkinson's disease. These results show that the inhibition of AAK1 activity is useful in treating schizophrenia, cognitive deficit in schizophrenia, Parkinson's disease, neuropathic pain, bipolar disorder and Alzheimer's disease.

Viruses enter cells via a variety of ways, such as endocytosis and membrane fusion. Endocytosis is the primary entry mechanism for most viruses, among which clathrin-mediated endocytosis is the predominant pathway. Viruses such as vesicular stomatitis virus (VSV), influenza A virus (IAV) and Crimean-Congo haemorrhagic fever virus (CCHFV) enter cells via clathrin-dependent pathways. Studies have found that multiple viruses, such as vesicular stomatitis virus (VSV), rabies virus (RABV) and hepatitis C virus (HCV), depend on AAK1 for their infection processes. These results indicate that the inhibition of AAK1 activity may have a potential therapeutic effect on viral infection-related diseases.

### Summary of the Invention

The present invention provides a compound represented by general formula (I), or a stereoisomer or a pharmaceutically acceptable salt thereof, which exhibits an inhibitory effect on AAK1, can inhibit cell proliferation, possesses good pharmacokinetic characteristics, high bioavailability, good safety, high selectivity and low toxicity and side effects, and has the advantages of oral administration, rapid absorption, high clearance, etc. Moreover, it has surprisingly been found that the compound of the present invention has a good brain penetrability.

An objective of the present invention is to provide a compound represented by general formula (I), (IIA) or (IIB), or a stereoisomer or a pharmaceutically acceptable salt thereof: 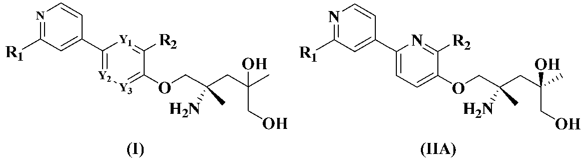 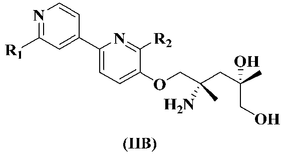 wherein:
Y₁, Y₂ and Y₃ are each independently CRₐ or N;
Rₐ is hydrogen, deuterium, halogen, hydroxyl, cyano, amino, C₁₋₆ alkyl, C₁₋₆ deuteroalkyl, C₁₋₆ haloalkyl, C₁₋₆ alkoxy, C₁₋₆ alkylthio, C₁₋₆ alkylamino, C₃₋₈ cycloalkyl, 3- to 8-membered heterocycloalkyl containing 1-3 heteroatoms selected from N, O or S, C₆₋₁₀ aryl, or 5- to 10-membered heteroaryl containing 1-3 heteroatoms selected from N, O or S;
R₁ and R₂ are each independently hydrogen, deuterium, halogen, cyano, amino, mercapto, carbamoyl, C₁₋₆ alkyl, C₁₋₆ deuteroalkyl, C₁₋₆ haloalkyl, C₁₋₆ alkoxy, C₁₋₆ alkylthio, C₁₋₆ alkylamino, C₁₋₆ hydroxyalkyl, halogenated C₁₋₆ alkoxy, deuterated C₁₋₆ alkoxy, C₃₋₈ cycloalkyl, 3- to 8-membered heterocycloalkyl containing 1-3 heteroatoms selected from N, O or S, C₆₋₁₀ aryl, 5- to 10-membered heteroaryl containing 1-3 heteroatoms selected from N, O or S, -C(O)C₁₋₆ alkyl, - NHC(O)C₁₋₆ alkyl, -NHC(O)OC₁₋₆ alkyl, -NHC(O)NHC₁₋₆ alkyl, -NHC(O)C₃₋₈ cycloalkyl, -NHC(O)OC₃₋₈ cycloalkyl, -NHC(O)C₄₋₆ heterocycloalkyl or - NHC(O)OC₄₋₆ heterocycloalkyl;
and the following compounds are excluded:

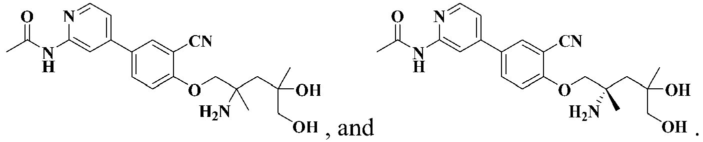

In some embodiments, Y₁, Y₂ and Y₃ are each independently CH or N; in some embodiments, Y₁, Y₂ and Y₃ are each independently CH or N, and Y₁, Y₂ and Y₃ are not CH simultaneously; in some embodiments, Y₁ is N, Y₂ and Y₃ are CH, or Y₂ is N, Y₁ and Y₃ are CH, or Y₃ is N, Y₁ and Y₂ are CH.

In some embodiments, Rₐ is hydrogen, halogen or C₁₋₆ alkyl; in some embodiments, Rₐ is hydrogen, halogen or C₁₋₃ alkyl; in some embodiments, Rₐ is hydrogen.

In some embodiments, R₁ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, - NHC(O)OC₁₋₆ alkyl or -NHC(O)NHC₁₋₆ alkyl; in some embodiments, R₁ is C₁₋₃ haloalkyl or -NHC(O)OC₁₋₃ alkyl; in some embodiments, R₁ is -CH₂F, -CHF₂, - CHF₃, -NHC(O)OCH₃, -NHC(O)OCH₂CH₃ or -NHC(O)OCH₂CH₂CH₃; in some embodiments, R₁ is -CHF₂ or -NHC(O)OCH₃.

In some embodiments, R₂ is hydrogen, deuterium, halogen, cyano, C₁₋₆ alkyl, C₁₋₆ deuteroalkyl, C₁₋₆ haloalkyl or C₁₋₆ alkoxy; in some embodiments, R₂ is cyano, C₁₋₃ alkyl or C₁₋₃ haloalkyl; in some embodiments, R₂ is cyano, methyl or -CHF₂.

In some embodiments, the compound represented by general formula (I) is selected from: 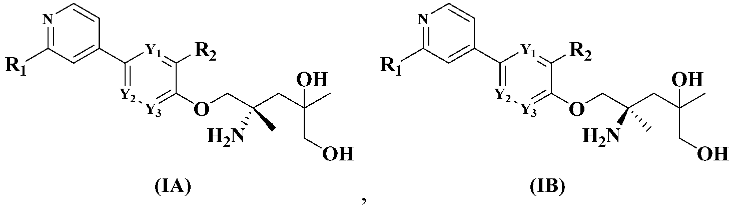 or a mixture thereof.

In a further embodiment of the present invention, the general formula (I) is further as represented by general formula (II): 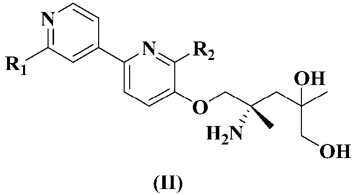 wherein
R₁ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, -NHC(O)OC₁₋₆ alkyl or -NHC(O)NHC₁₋₆ alkyl; in some embodiments, R₁ is C₁₋₆ haloalkyl or - NHC(O)OC₁₋₆ alkyl; in some embodiments, R₁ is C₁₋₃ haloalkyl or -NHC(O)OC₁₋₃ alkyl; in some embodiments, R₁ is -CHF₂ or -NHC(O)OCH₃;
R₂ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, -NHC(O)OC₁₋₆ alkyl or -NHC(O)NHC₁₋₆ alkyl; in some embodiments, R₂ is C₁₋₆ alkyl, cyano or C₁₋₆ haloalkyl; in some embodiments, R₂ is C₁₋₆ alkyl or C₁₋₆ haloalkyl; in some embodiments, R₂ is C₁₋₃ alkyl or C₁₋₃ haloalkyl; in some embodiments, R₂ is methyl, -CH₂CH₂F, -CH₂CHF₂, -CH₂CF₃, -CH₂F, -CHF₂ or -CF₃; in some embodiments, R₂ is methyl or -CHF₂.

In a further embodiment of the present invention, the general formula (II) is further as represented by general formula (IIA) or general formula (IIB): 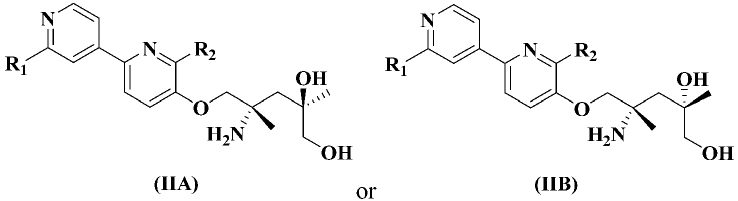 wherein
R₁ and R₂ are as defined in any of the above technical solutions.

In a further embodiment of the present invention, the compound represented by general formula (I), or the stereoisomer or pharmaceutically acceptable salt thereof is selected from the following compounds:

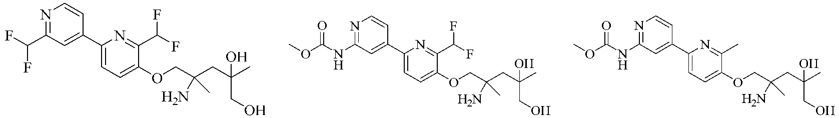

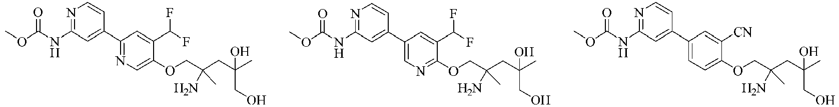

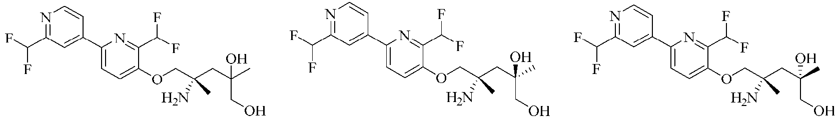

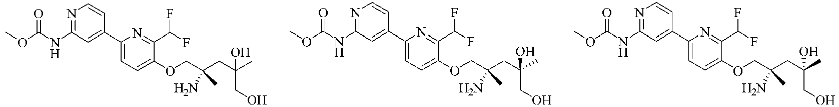

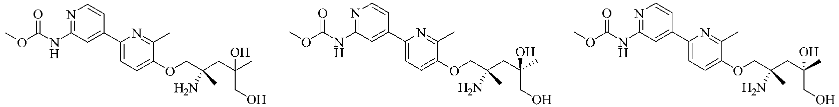

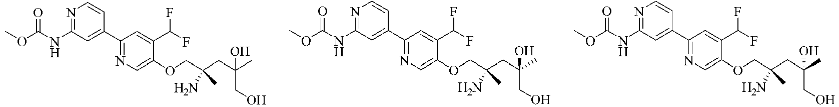

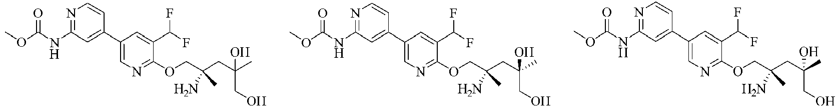

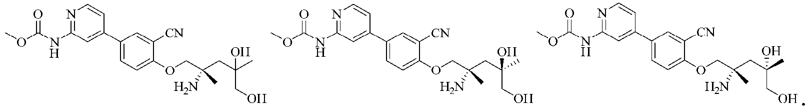

The present invention also provides a pharmaceutical composition comprising a therapeutically effective dose of the compound, or the stereoisomer or pharmaceutically acceptable salt thereof according to any one of the above, and one or more pharmaceutically acceptable carriers or excipients.

In a further embodiment of the present invention, the pharmaceutical composition comprises 1-1500 mg of the compound, or the stereoisomer or pharmaceutically acceptable salt thereof according to any one of the above, and one or more pharmaceutically acceptable carriers or excipients.

The present invention also provides the use of the compound, or the stereoisomer or pharmaceutically acceptable salt thereof according to any one of the above, or the above-described pharmaceutical composition in the preparation of a medicament, wherein preferably the medicament is a medicament for preventing and/or treating AAK1-mediated diseases.

In a further embodiment of the present invention, the AAK1-mediated disease is diabetic neuropathic pain or post-herpetic neuralgia.

The present invention also provides a method for treating a disease in a mammal, wherein the method comprises administering to a subject a therapeutically effective amount of the compound, or the stereoisomer or pharmaceutically acceptable salt thereof according to any one of the above, or the above-described pharmaceutical composition, wherein the therapeutically effective amount is preferably 1-1500 mg, and the disease is preferably diabetic neuropathic pain or post-herpetic neuralgia.

The "effective amount" or "therapeutically effective amount" described in the present application refers to administration of a sufficient amount of the compound disclosed in the present application that will alleviate to some extent one or more symptoms of the diseases or conditions being treated. In some embodiments, the outcome is the reduction and/or remission of signs, symptoms, or causes of the disease, or any other desired change in the biological system. For example, an "effective amount" in terms of the therapeutic use is an amount of the composition comprising the peptide compound, conjugate or the pharmaceutically acceptable salt thereof disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;

In some embodiments, the pharmaceutical composition or preparation of the present invention contains the above therapeutically effective amount of the compound, or the stereoisomer or pharmaceutically acceptable salt thereof according to any one of the above.

The present invention further relates to a pharmaceutical composition or pharmaceutical preparation, wherein the pharmaceutical composition or pharmaceutical preparation comprises a therapeutically effective amount of the compound, or the stereoisomer or pharmaceutically acceptable salt thereof according to any one of the above, and one or more pharmaceutically acceptable carriers or excipients. The pharmaceutical composition can be in a unit preparation form (the amount of the active ingredient in the unit preparation is also referred to as the "preparation strength"). In some embodiments, the pharmaceutical composition includes, but is not limited to, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg or 1500 mg of the compound, or the stereoisomer or pharmaceutically acceptable salt thereof according to any one of the above.

The present invention further relates to a method for treating a disease in a mammal, wherein the method comprises administering to a subject a therapeutically effective amount of the compound, or the stereoisomer or pharmaceutically acceptable salt thereof according to any one of the above, and one or more pharmaceutically acceptable carriers or excipients, wherein the therapeutically effective amount is preferably 1-1500 mg, and the disease is preferably neuropathic pain, more preferably diabetic neuropathic pain or post-herpetic neuralgia.

The present invention further relates to a method for treating a disease in a mammal, wherein the method comprises administering to a subject the compound, or the stereoisomer or pharmaceutically acceptable salt thereof according to any one of the above of the present invention, as a drug, and one or more pharmaceutically acceptable carriers or excipients at a daily dose of 1-1500 mg/day, wherein the daily dose may be a single dose or divided doses. In some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day. In some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 300 mg/day, 400 mg/day, 600 mg/day, 800 mg/day, 1000 mg/day, 1200 mg/day, 1400 mg/day, and 1500 mg/day.

The present invention relates to a kit, which may comprise a composition in a single-dose or multi-dose form, wherein the kit comprises the compound, or the stereoisomer or pharmaceutically acceptable salt thereof according to any one of the above of the present invention, and the amount of the compound, or the stereoisomer or pharmaceutically acceptable salt thereof according to the present invention is the same as that in the above-described pharmaceutical composition.

In the present invention, the amount of the compound, or the stereoisomer or the pharmaceutically acceptable salt thereof according to the present invention is calculated in the form of a free base in each case.

The term "preparation strength" refers to the weight of the active ingredient contained in each vial, tablet or other unit preparation.

### Synthetic route

Those skilled in the art would have been able to prepare the compounds of the present invention by means of combining the documents WO 2023284838, WO 2017059085, WO 2017059080, and WO 2015153720 and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3 527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups", John Wiley & Sons, in 73 volumes.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

### Detailed Description of the Invention

Unless defined otherwise, all technical and scientific terms used herein have the same meaning as commonly understood by those of ordinary skill in the field to which the present invention belongs. In case of conflict, the definitions provided in the present application will control. When a trade name appears herein, it is intended to refer to the corresponding commodity or an active ingredient thereof. All patents, published patent applications, and publications cited herein are incorporated herein by reference.

The term "alkyl" refers to a saturated, straight or branched aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl preferably has 1 to 12 carbon atoms (i.e., C₁₋₁₂ alkyl), more preferably 1 to 8 carbon atoms (i.e., C₁₋₈ alkyl), further preferably 1 to 6 carbon atoms (i.e., C₁₋₆ alkyl), and most preferably 1 to 3 carbon atoms (i.e., C₁₋₃ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When the alkyl is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the alkyl is substituted with a substituent, the substituent is not further substituted.

The term "cycloalkyl" refers to a saturated or partially unsaturated monocyclic cyclic hydrocarbon substituent (i.e., monocyclic cycloalkyl) or polycyclic cyclic hydrocarbon substituent (i.e., polycyclic cycloalkyl) having 3 to 20 (e.g., 3, 4, 5, 6, 7, 8, 9, 10, 11, 12, 13, 14, 15, 16, 17, 18, 19, or 20) carbon atoms (i.e., C₃₋₂₀ cycloalkyl). The cycloalkyl preferably has 3 to 12 carbon atoms (i.e., C₃₋₁₂ cycloalkyl), more preferably 3 to 8 carbon atoms (i.e., C₃₋₈ cycloalkyl), further preferably 3 to 6 carbon atoms (i.e., C₃₋₆ cycloalkyl), and most preferably 3 to 5 carbon atoms (i.e., C₃₋₅ cycloalkyl), or 5 to 6 carbon atoms (i.e., C₃₋₅ cycloalkyl). Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Non-limiting examples of the polycyclic cycloalkyl include: spirocycloalkyl, fused cycloalkyl and bridged cycloalkyl.

The term "spirocycloalkyl" refers to a polycyclic group with monocyclic rings sharing one carbon atom (called a spiro atom). It may contain one or more double bonds, but none of the rings has a fully conjugated π electron system. The spirocycloalkyl has 5 to 20 (e.g., 5, 6, 7, 8, 9, 10, 11, 12, 13, 14, 15, 16, 17, 18, 19 or 20) ring atoms (i.e., C₅₋₂₀ spirocycloalkyl). The spirocycloalkyl preferably has 6 to 14 ring atoms (i.e., C₆₋₁₄ spirocycloalkyl), more preferably 7 to 10 ring atoms (i.e., C₇₋₁₀ spirocycloalkyl). According to the number of shared spiro atoms between rings, the spirocycloalkyl is divided into monospirocycloalkyl, dispirocycloalkyl or polyspirocycloalkyl, preferably monospirocycloalkyl or dispirocycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl.

The term "fused cycloalkyl" refers to an all-carbon polycyclic group in which each ring in the system shares an adjacent pair of carbon atoms with other rings in the system, having 5 to 20 (e.g., 5, 6, 7, 8, 9, 10, 11, 12, 13, 14, 15, 16, 17, 18, 19 or 20) ring atoms (i.e., C₅₋₂₀ fused cycloalkyl). It may contain one or more double bonds, but none of the rings has a fully conjugated π electron system. The fused cycloalkyl preferably has 6 to 14 ring atoms (i.e., C₆₋₁₄ fused cycloalkyl), more preferably 7 to 10 ring atoms (i.e., C₇₋₁₀ fused cycloalkyl). According to the number of constituent rings, it can be divided into bicyclic, tricyclic, tetracyclic or polycyclic fused cycloalkyl, preferably bicyclic fused cycloalkyl or tricyclic fused cycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl.

The term "bridged cycloalkyl" refers to an all-carbon polycyclic group in which any two rings share two carbon atoms that are not directly connected to each other, having 5 to 20 (e.g., 5, 6, 7, 8, 9, 10, 11, 12, 13, 14, 15, 16, 17, 18, 19 or 20) ring atoms (i.e., C₅₋₂₀ bridged cycloalkyl). It contains one or more double bonds, but none of the rings has a fully conjugated π electron system. The bridged cycloalkyl preferably has 6 to 14 ring atoms (i.e., C₆₋₁₄ bridged cycloalkyl), more preferably 7 to 10 ring atoms (i.e., C₇₋₁₀ bridged cycloalkyl). According to the number of constituent rings, it can be divided into bicyclic, tricyclic, tetracyclic or polycyclic bridged cycloalkyl, preferably bicyclic bridged cycloalkyl or tricyclic bridged cycloalkyl.

The cycloalkyl can be fused to an aryl, heteroaryl or heterocycloalkyl ring, wherein the ring connected to the parent structure is cycloalkyl.

The cycloalkyl can be optionally substituted or unsubstituted. When the cycloalkyl is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the cycloalkyl is substituted with a substituent, the substituent is not further substituted.

The term "heterocycloalkyl" refers to a saturated or partially unsaturated monocyclic heterocyclic hydrocarbon substituent (i.e., monocyclic heterocycloalkyl) or polycyclic heterocyclic hydrocarbon substituent (i.e., polycyclic heterocycloalkyl) having 3 to 20 (e.g., 3, 4, 5, 6, 7, 8, 9, 10, 11, 12, 13, 14, 15, 16, 17, 18, 19 or 20) ring atoms (i.e., 3- to 20-membered heterocycloalkyl), wherein one or more (e.g., 1, 2, 3 or 4) of the ring atoms are heteroatoms selected from nitrogen, oxygen, P(O)ₘ and S(O)ₙ (wherein m and n are integers of 0-2), with no -O-O-, -O-S-, or -S-S- present in the ring, and the remaining ring atoms are carbon. The heterocycloalkyl preferably has 3 to 12 ring atoms (i.e., 3- to 12-membered heterocycloalkyl) containing 1-4 heteroatoms selected from N, O, and S atoms, more preferably has 3 to 8 ring atoms (i.e., 3- to 8-membered heterocycloalkyl) containing 1-4, 1-3, or 1-2 heteroatoms selected from N, O, and S atoms, further preferably has 3 to 6 ring atoms (i.e., 3- to 6-membered heterocycloalkyl) containing 1-4, 1-3, or 1-2 heteroatoms selected from N, O, and S atoms, and most preferably has 5 to 6 ring atoms (i.e., 5- to 6-membered heterocycloalkyl) containing 1-4, 1-3, or 1-2 heteroatoms selected from N, O, and S atoms. Non-limiting examples of the monocyclic heterocycloalkyl include: azetidinyl, oxetanyl, thietanyl, pyrrolidinyl, imidazolidinyl, tetrahydrofuryl, tetrahydrothienyl, tetrahydropyranyl, dihydroimidazolyl, dihydrofuryl, dihydropyrazolyl, piperidinyl, piperazinyl, morpholinyl, 1,3-dioxolanyl, 2,2-difluoro-1,3-dioxolanyl, cyclopentanonyl, 2,2-difluorocyclopentanonyl, azepanyl, oxolanyl or azacyclopentyl. Non-limiting examples of the polycyclic heterocycloalkyl include: spiroheterocycloalkyl, fused heterocycloalkyl and bridged heterocycloalkyl.

The term "spiroheterocycloalkyl" refers to a polycyclic heterocyclyl group in which monocyclic rings share one atom (called a spiro atom), having 5 to 20 (e.g., 5, 6, 7, 8, 9, 10, 11, 12, 13, 14, 15, 16, 17, 18, 19 or 20) ring atoms (i.e., 5- to 20-membered spiroheterocycloalkyl), wherein one or more (e.g., 1, 2, 3 or 4) of the ring atoms are heteroatoms selected from nitrogen, oxygen, P(O)ₘ and S(O)ₙ (wherein m and n are integers of 0-2), with no -O-O-, -O-S-, or -S-S- present in the ring, and the remaining ring atoms are carbon. It may contain one or more double bonds, but none of the rings has a fully conjugated π electron system. The spiroheterocycloalkyl preferably has 6 to 14 ring atoms (i.e., 6- to 14-membered spiroheterocycloalkyl), more preferably 7 to 10 ring atoms (i.e., 7- to 10-membered spiroheterocycloalkyl). According to the number of shared spiro atoms between rings, the spiroheterocycloalkyl is divided into monospiroheterocycloalkyl, dispiroheterocycloalkyl or polyspiroheterocycloalkyl, preferably monospiroheterocycloalkyl or dispiroheterocycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocycloalkyl.

The term "fused heterocycloalkyl" refers to a polycyclic heterocycloalkyl group in which each ring in the system shares an adjacent pair of atoms with other rings in the system, having 5 to 20 (e.g., 5, 6, 7, 8, 9, 10, 11, 12, 13, 14, 15, 16, 17, 18, 19 or 20) ring atoms (i.e., 5- to 20-membered fused heterocycloalkyl), wherein one or more (e.g., 1, 2, 3 or 4) of the ring atoms are heteroatoms selected from nitrogen, oxygen, P(O)ₘ and S(O)ₙ (wherein m and n are integers of 0-2), with no - O-O-, -O-S-, or -S-S- present in the ring, and the remaining ring atoms are carbon. It may contain one or more double bonds, but none of the rings has a fully conjugated π electron system. The fused heterocycloalkyl preferably has 6 to 14 ring atoms (i.e., 6- to 14-membered fused heterocycloalkyl), more preferably 7 to 10 ring atoms (i.e., 7- to 10-membered fused heterocycloalkyl). According to the number of constituent rings, it can be divided into bicyclic, tricyclic, tetracyclic or polycyclic fused heterocycloalkyl, preferably bicyclic fused heterocycloalkyl or tricyclic fused heterocycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocycloalkyl.

The term "bridged heterocycloalkyl" refers to a polycyclic heterocycloalkyl group in which any two rings share two atoms that are not directly connected to each other, having 5 to 20 (e.g., 5, 6, 7, 8, 9, 10, 11, 12, 13, 14, 15, 16, 17, 18, 19 or 20) ring atoms (i.e., 5- to 20-membered bridged heterocycloalkyl), wherein one or more (e.g., 1, 2, 3 or 4) of the ring atoms are heteroatoms selected from nitrogen, oxygen, P(O)ₘ and S(O)ₙ (wherein m and n are integers of 0-2), with no -O-O-, -O-S-, or -S-S- present in the ring, and the remaining ring atoms are carbon. It may contain one or more double bonds, but none of the rings has a fully conjugated π electron system. The bridged heterocycloalkyl preferably has 6 to 14 ring atoms (i.e., 6- to 14-membered bridged heterocycloalkyl), more preferably 7 to 10 ring atoms (i.e., 7- to 10-membered bridged heterocycloalkyl). According to the number of constituent rings, it can be divided into bicyclic, tricyclic, tetracyclic or polycyclic bridged heterocycloalkyl, preferably bicyclic bridged heterocycloalkyl or tricyclic bridged heterocycloalkyl.

The heterocycloalkyl can be fused to an aryl, heteroaryl or cycloalkyl ring, wherein the ring connected to the parent structure is heterocycloalkyl.

The heterocycloalkyl can be optionally substituted or unsubstituted. When the heterocycloalkyl is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the heterocycloalkyl is substituted with a substituent, the substituent is not further substituted.

The term "aryl" refers to an all-carbon monocyclic group (i.e., monocyclic aryl) or fused polycyclic group (i.e., polycyclic aryl) having a conjugated π-electron system and having 6 to 14 (e.g., 6, 7, 8, 9, 10, 11, 12, 13 or 14) carbon atoms (i.e., C₆₋₁₄ aryl). The aryl preferably has 6 to 12 carbon atoms (i.e., C₆₋₁₂ aryl), more preferably 6 to 10 carbon atoms (i.e., C₆₋₁₀ aryl), further preferably phenyl or naphthyl, and most preferably phenyl. The aryl is monocyclic aryl, such as phenyl. Non-limiting examples of the polycyclic aryl include: naphthyl, anthryl, phenanthryl, etc.

The aryl can be fused to a heteroaryl, heterocycloalkyl or cycloalkyl ring, wherein the ring connected to the parent structure is an aryl ring. The aryl can be optionally substituted or unsubstituted. When the aryl is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the aryl is substituted with a substituent, the substituent is not further substituted.

The term "heteroaryl" refers to a monocyclic heteroaryl group (i.e., monocyclic heteroaryl) or fused polycyclic heteroaryl group (i.e., polycyclic heteroaryl) having a conjugated π-electron system and having 5 to 14 (e.g., 5, 6, 7, 8, 9, 10, 11, 12, 13 or 14) ring atoms (i.e., 5- to 14-membered heteroaryl), wherein one or more (e.g., 1, 2, 3 or 4) of the ring atoms are heteroatoms selected from nitrogen, oxygen, P(O)ₘ and S(O)ₙ (wherein m and n are integers of 0-2), preferably heteroatoms selected from nitrogen, oxygen, or sulphur, with no -O-O-, -O-S-, or -S-S- present in the ring, and the remaining ring atoms are carbon. The heteroaryl preferably has 5 to 10 ring atoms (i.e., 5- to 10-membered heteroaryl). The monocyclic heteroaryl is preferably heteroaryl having 5 to 6 ring atoms (i.e., 5- to 6-membered heteroaryl), and non-limiting examples include: furyl, pyranyl, thienyl, thiazolyl, isothiazolyl, oxazolyl, isoxazolyl, oxadiazolyl, thiadiazolyl, imidazolyl, pyrazolyl, triazolyl, tetrazolyl, pyrrolyl, pyridyl, pyrimidyl, pyridonyl, pyrazinyl, pyridazinyl, etc. The polycyclic heteroaryl, preferably 5- to 6-membered heteroaryl fused 5- to 6-membered heteroaryl, 5- to 10-membered heteroaryl fused C₆₋₁₀ aryl or C₆₋₁₀ aryl fused 5- to 10-membered heteroaryl, further preferably 5- to 6-membered heteroaryl fused 5- to 6-membered heteroaryl, 5- to 6-membered heteroaryl fused phenyl or phenyl fused 5- to 6-membered heteroaryl, and non-limiting examples include: indolyl, indazolyl, quinolyl, isoquinolyl, quinoxalinyl, phthalazinyl, benzimidazolyl, benzothienyl, thienophenyl, quinazolinyl, benzothiazolyl, carbazolyl, thienopyridyl, pyridothienyl, pyridopyrrolyl, etc.

The heteroaryl can be fused to an aryl, heterocycloalkyl or cycloalkyl ring, wherein the ring connected to the parent structure is a heteroaryl ring. The heteroaryl can be optionally substituted or unsubstituted. When the heteroaryl is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the heteroaryl is substituted with a substituent, the substituent is not further substituted.

The term "alkoxy" refers to -O-(alkyl) or -O-(unsubstituted cycloalkyl), wherein the alkyl and cycloalkyl are as defined above, having 1 to 10 (e.g., 1, 2, 3, 4, 5, 6, 7, 8, 9 or 10) carbon atoms (i.e., C₁₋₁₀ alkoxy). The alkoxy preferably has 1 to 8 carbon atoms (i.e., C₁₋₈ alkoxy), more preferably 1 to 6 carbon atoms (i.e., C₁₋₆ alkoxy), and most preferably 1 to 3 carbon atoms (i.e., C₁₋₃ alkoxy). Non-limiting examples include: methoxy, ethoxy, propoxy, butoxy, cyclopropoxy, cyclobutoxy, cyclopentoxy, cyclohexoxy, etc. The alkoxy can be optionally substituted or unsubstituted. When the alkoxy is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the alkoxy is substituted with a substituent, the substituent is not further substituted.

The term "alkylthio" refers to -S-(alkyl) or -S-(unsubstituted cycloalkyl), wherein the alkyl and cycloalkyl are as defined above, having 1 to 10 (e.g., 1, 2, 3, 4, 5, 6, 7, 8, 9 or 10) carbon atoms (i.e., C₁₋₁₀ alkylthio). The alkylthio preferably has 1 to 8 carbon atoms (i.e., C₁₋₈ alkylthio), more preferably 1 to 6 carbon atoms (i.e., C₁₋₆ alkylthio), and most preferably 1 to 3 carbon atoms (i.e., C₁₋₃ alkylthio). Non-limiting examples include: methylthio, ethylthio, propylthio, butylthio, cyclopropylthio, cyclobutylthio, cyclopentylthio, cyclohexylthio, etc. The alkylthio can be optionally substituted or unsubstituted. When the alkylthio is substituted, the substitution may occur at any available point of attachment, and the substituent is preferably one or more groups independently selected from deuterium, alkyl, alkenyl, alkynyl, alkoxy, alkylthio, halogen, mercapto, hydroxyl, nitro, amino, cyano, carboxyl, oxo, cycloalkyl, heterocycloalkyl, aryl or heteroaryl. When the alkylthio is substituted with a substituent, the substituent is not further substituted.

The term "halo" or "halogen" or "halogenated" should be understood as denoting fluorine (F), chlorine (Cl), bromine (Br), or iodine (I) atoms, preferably fluorine, chlorine, or bromine atoms.

The term "haloalkyl" refers to alkyl substituted with one or more halogen, wherein the alkyl is as defined above. Non-limiting examples include: fluoromethyl, chloromethyl, bromomethyl, iodomethyl, difluoromethyl, chlorofluoromethyl, dichloromethyl, bromofluoromethyl, trifluoromethyl, chlorodifluoromethyl, dichlorofluoromethyl, trichloromethyl, bromodifluoromethyl, bromochlorofluoromethyl, dibromofluoromethyl, 2-fluoroethyl, 2-chloroethyl, 2-bromoethyl, 2,2-difluoroethyl, 2-chloro-2-fluoroethyl, 2,2-dichloroethyl, 2-bromo-2-fluoroethyl, 2,2,2-trifluoroethyl, 2-chloro-2,2-difluoroethyl, 2,2-dichloro-2-fluoroethyl, 2,2,2-trichloroethyl, 2-bromo-2,2-difluoroethyl, 2-bromo-2-chloro-2-fluoroethyl, 2-bromo-2,2-dichloroethyl, 1,1,2,2-tetrafluoroethyl, pentafluoroethyl, 1-chloro-1,2,2,2-tetrafluoroethyl, 2-chloro-1,1,2,2-tetrafluoroethyl, 1,2-dichloro-1,2,2-trifluoroethyl, 2-bromo-1,1,2,2-tetrafluoroethyl, etc., preferably fluoromethyl, difluoromethyl, trifluoromethyl, 2-fluoroethyl, 2-chloroethyl, 2-bromoethyl, 2,2-difluoroethyl.

The term "haloalkoxy" refers to alkoxy substituted with one or more halogen, wherein the alkoxy is as defined above. Non-limiting examples include: fluoromethoxy, chloromethoxy, bromomethoxy, iodomethoxy, difluoromethoxy, chlorofluoromethoxy, dichloromethoxy, bromofluoromethoxy, trifluoromethoxy, chlorodifluoromethoxy, dichlorofluoromethoxy, trichloromethoxy, bromodifluoromethoxy, bromochlorofluoromethoxy, dibromofluoromethoxy, etc.; preferably fluoromethoxy, difluoromethoxy, trifluoromethoxy, 2-fluoroethoxy, 2-chloroethoxy, 2-bromoethoxy, 2,2-difluoroethoxy, 2-chloro-2-fluoroethoxy, 2,2-dichloroethoxy, 2-bromo-2-fluoroethoxy, 2,2,2-trifluoroethoxy, 2-chloro-2,2-difluoroethoxy, 2,2-dichloro-2-fluoroethoxy, 2,2,2-trichloroethoxy, 2-bromo-2,2-difluoroethoxy, 2-bromo-2-chloro-2-fluoroethoxy, 2-bromo-2,2-dichloroethoxy, 1,1,2,2-tetrafluoroethoxy, pentafluoroethoxy, 1-chloro-1,2,2,2-tetrafluoroethoxy, 2-chloro-1,1,2,2-tetrafluoroethoxy, 1,2-dichloro-1,2,2-trifluoroethoxy, 2-bromo-1,1,2,2-tetrafluoroethoxy, preferably fluoromethoxy, difluoromethoxy, trifluoromethoxy, 2-fluoroethoxy, 2-chloroethoxy, 2-bromoethoxy or 2,2-difluoroethoxy.

The term "mercapto" refers to -SH.

The term "hydroxyl" refers to -OH.

The term "nitro" refers to -NO₂.

The term "amino" refers to -NH₂.

The term "cyano" refers to -CN.

The term "carboxyl" refers to -C(O)OH.

The term "oxo" or "oxo group" refers to =O.

The term "carbonyl" refers to C=O.

The term "carbamoyl" refers to -C(O)NH₂.

The term "sulphonyl" refers to -S(O)₂.

The term "deuteroalkyl" refers to alkyl substituted with one or more deuterium, wherein the alkyl is as defined above.

The term "deuteroalkoxy" refers to alkoxy substituted with one or more deuterium, wherein the alkoxy is as defined above.

The term "haloalkoxy" refers to alkoxy substituted with one or more halogen, wherein the alkoxy is as defined above.

The term "hydroxyalkyl" refers to alkyl substituted with one or more hydroxyl, wherein the alkyl is as defined above.

The term "alkylamino" refers to alkyl-NH-, wherein the alkyl is as defined above.

The terms "include", "comprise", "have", "contain", or "involve" and other variations thereof as used herein are inclusive or open-ended and do not exclude other unlisted elements or method steps. It should be understood by those skilled in the art that the above terms such as "include" encompass the meaning of "consisting of".

The term "one or more" or similar expression "at least one" may mean, for example, 1, 2, 3, 4, 5, 6, 7, 8, 9, 10, or more.

Where a lower limit and an upper limit of a numerical range are disclosed, any numerical value and any subrange falling within the range are specifically disclosed. In particular, each range of values disclosed herein should be understood as encompassing each individual value and each subrange included within the broader range.

Herein, "Z" and "-Z-" both refer to the same specific group and can be used interchangeably.

The expression "m-n" used herein refers to the range from m to n, as well as the subranges composed of each individual point value within the range, and each individual point value therein. For example, the expression "C₂-C₈" or "C₂₋₈" encompasses the range of 2-8 carbon atoms, and should be understood to also encompass any subranges and each individual point value therein, such as C₂-C₅, C₃-C₄, C₂-C₆, C₃-C₆, C₄-C₆, C₄-C₇, and C₄-C₈, as well as C₂, C₃, C₄, C₅, C₆, C₇, and C₈. For example, the expression "C₃-C₁₀" or "C₃₋₁₀" should also be understood in a similar manner, encompassing any subranges and individual point values contained therein, such as C₃-C₉, C₆-C₉, C₆-C₈, C₆-C₇, C₇-C₁₀, C₇-C₉, C₇-C₈, and C₈-C₉, as well as C₃, C₄, C₅, C₆, C₇, C₈, C₉, and C₁₀. For another example, the expression "C₁-C₆" or "C₁₋₆" encompasses the range of 1-6 carbon atoms, and should be understood to also encompass any subranges and each individual point value therein, such as C₂-C₅, C₃-C₄, C₁-C₂, C₁-C₃, C₁-C₄, C₁-C₅, and C₁-C₆, as well as C₁, C₂, C₃, C₄, C₅, and C₆. For another example, the expression "three- to ten-membered" should be understood to encompass any subranges and each individual point value therein, such as three- to five-membered, three- to six-membered, three- to seven-membered, three- to eight-membered, four- to five-membered, four- to six-membered, four- to seven-membered, four- to eight-membered, five- to seven-membered, five- to eight-membered, six- to seven-membered, six- to eight-membered, and nine- to ten-membered, as well as three-, four-, five-, six-, seven-, eight-, nine-, or ten-membered. Other similar expressions herein should also be understood in a similar manner.

Different expressions used herein such as "X is selected from A, B or C", "X is selected from A, B and C", "X is A, B or C", and "X is A, B and C" all express the same meaning, that is, X can be any one or more of A, B, and C.

The terms "optional" or "optionally" mean that the subsequently described event or circumstance may or may not occur, and the description includes the occurrence and the non-occurrence of the event or circumstance. For example, "cycloalkyl optionally substituted with alkyl" means that the alkyl may but not necessarily be present, and the description includes the case where the cycloalkyl is substituted with alkyl and the case where the cycloalkyl is not substituted with alkyl.

The terms "substitution" and "substituted" means that one or more (e.g., one, two, three, or four) hydrogen atoms on a designated atom are replaced by substituents selected from the indicated groups, provided that the normal valence of the designated atom under the existing circumstances is not exceeded and the substitution results in a stable compound. Combinations of substituents and/or variables are permitted only when such combinations result in stable compounds. When a substituent is described as being absent, it should be understood that the substituent can be one or more hydrogen, provided that the structure allows the compound to achieve a stable state. When it is described that each carbon atom in a group can be optionally replaced by a heteroatom, the normal valence of all atoms in the group under the existing circumstances shall not be exceeded and a stable compound is formed thereby.

If a substituent is described as being "optionally substituted with ....", the substituent may be unsubstituted or substituted. If an atom or a group is described as being optionally substituted with one or more substituents from a list, one or more hydrogen on the atom or the group may be replaced by optional substituents that are independently selected. When the substituent is oxo (i.e., =O), it means that two hydrogen atoms are replaced. When the substituent is hydrogen, it may also mean that the corresponding group is "unsubstituted". Unless specified otherwise, the attachment point of a substituent as used herein may be at any suitable position of the substituent.

When a bond to a substituent is shown to cross a ring (a bond connecting two atoms in a ring), such substituent may be bonded to any of the substitutable ring-forming atoms in that ring.

When any variable (e.g., R) and any marked variable (e.g., R₁, R₂, R₃, R₄, R₅, R₆, and R₇) appear more than once in the constitution or structure of a compound, its definition upon each occurrence in each case is independent. For example, if a group is substituted with 0, 1, 2, 3 or 4 R substituents, the group may optionally be substituted with up to four R substituents, and each R substituent in each case is independently selected.

The compounds of the present invention may exist in specific geometric or stereoisomeric forms. All such compounds of the present invention include cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present invention. Additional asymmetric carbon atoms may be present in the substituents of the compounds of the present invention. All such isomers, as well as mixtures thereof, are included within the scope of the present invention. In certain embodiments, preferred compounds are those isomeric compounds that exhibit superior biological activity. Purified or partially purified isomers and stereoisomers, or racemic mixtures or diastereomeric mixtures of the compounds of the present invention are also included within the scope of the present invention. Purification and isolation of such substances can be achieved by standard techniques known in the art.

The hydrogen atoms according to the present invention can be replaced by the isotope deuterium thereof, and any hydrogen atom in the example compounds involved in the present invention can also be replaced by a deuterium atom.

The compounds of the present invention include all suitable isotopic derivatives thereof. The term "isotopic derivative" refers to a compound in which at least one atom is replaced by an atom having the same atomic number but a different atomic mass. Examples of isotopes that can be introduced into the compounds of the present disclosure include stable and radioactive isotopes of hydrogen, carbon, nitrogen, oxygen, phosphorus, sulphur, fluorine, chlorine, bromine, iodine, etc., such as ²H (deuterium, D),³H (tritium, T), ¹¹C, ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, preferably deuterium.

Deuterated drugs offer advantages over their non-deuterated counterparts, such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom can be independently replaced by a deuterium atom, where such deuterium replacement can be partial or complete, and partial deuterium replacement means that at least one hydrogen atom is replaced by at least one deuterium atom.

In the compounds of the present invention, when a position is specifically designated as deuterium (D), it should be understood that the position has a deuterium abundance at least 1000 times greater than the natural abundance of deuterium (which is 0.015%) (i.e., at least 15% deuterium incorporation). In some embodiments, the deuterium abundance in each designated deuterium atom is at least 1000 times greater than the natural abundance of deuterium (i.e., at least 15% deuterium incorporation). In some embodiments, the deuterium abundance in each designated deuterium atom is at least 2000 times greater than the natural abundance of deuterium (i.e., at least 30% deuterium incorporation). In some embodiments, the deuterium abundance in each designated deuterium atom is at least 3000 times greater than the natural abundance of deuterium (i.e., at least 45% deuterium incorporation). In some embodiments, the deuterium abundance in each designated deuterium atom is at least 3340 times greater than the natural abundance of deuterium (i.e., at least 50.1% deuterium incorporation). In some embodiments, the deuterium abundance in each designated deuterium atom is at least 3500 times greater than the natural abundance of deuterium (i.e., at least 52.5% deuterium incorporation). In some embodiments, the deuterium abundance in each designated deuterium atom is at least 4000 times greater than the natural abundance of deuterium (i.e., at least 60% deuterium incorporation). In some embodiments, the deuterium abundance in each designated deuterium atom is at least 4500 times greater than the natural abundance of deuterium (i.e., at least 67.5% deuterium incorporation). In some embodiments, the deuterium abundance in each designated deuterium atom is at least 5000 times greater than the natural abundance of deuterium (i.e., at least 75% deuterium incorporation). In some embodiments, the deuterium abundance in each designated deuterium atom is at least 5500 times greater than the natural abundance of deuterium (i.e., at least 82.5% deuterium incorporation). In some embodiments, the deuterium abundance in each designated deuterium atom is at least 6000 times greater than the natural abundance of deuterium (i.e., at least 90% deuterium incorporation). In some embodiments, the deuterium abundance in each designated deuterium atom is at least 6333.3 times greater than the natural abundance of deuterium (i.e., at least 95% deuterium incorporation). In some embodiments, the deuterium abundance in each designated deuterium atom is at least 6466.7 times greater than the natural abundance of deuterium (i.e., at least 97% deuterium incorporation). In some embodiments, the deuterium abundance in each designated deuterium atom is at least 6600 times greater than the natural abundance of deuterium (i.e., at least 99% deuterium incorporation). In some embodiments, the deuterium abundance in each designated deuterium atom is at least 6633.3 times greater than the natural abundance of deuterium (i.e., at least 99.5% deuterium incorporation).

The term "pharmaceutically acceptable" substance refers to a substance that is, within the scope of sound medical judgment, suitable for use in contact with patient tissues without undue toxicity, irritation, allergic response, etc., has a reasonable benefit-to-risk ratio, and is effective for its intended use.

The term "pharmaceutically acceptable salt" refers to a salt of the compound of the present invention, which is safe and effective when used in mammals, and exhibits the intended biological activity.

The term "pharmaceutical composition" refers to a composition containing one or more compounds described in the present invention or physiologically/pharmaceutically acceptable salts or prodrugs thereof together with other components, such as physiologically/pharmaceutically acceptable carriers or excipients. The purpose of the pharmaceutical compositions is to facilitate administration to living organisms and facilitate the absorption of active ingredients to exert biological activity.

The term "pharmaceutically acceptable carrier" refers to those substances that have no significant irritating effects on the organism and do not impair the bioactivity and properties of the active compound. "Pharmaceutically acceptable carriers" include, but are not limited to, glidants, sweetening agents, diluents, preservatives, dyes/colourants, flavouring agents, surfactants, wetting agents, dispersing agents, disintegrants, stabilisers, solvents, or emulsifiers.

The terms "administration", "administering", etc., refer to methods that enable the delivery of a compound or composition to a desired site of biological action. These methods include, but are not limited to, oral or parenteral (including intraventricular, intravenous, subcutaneous, intraperitoneal, intramuscular, intravascular injection or infusion), topical, and rectal administration. In particular, injection or oral administration.

As used herein, the term "treatment" includes relieving, alleviating, or improving a disease or symptom; preventing other symptoms; improving or preventing the underlying metabolic factors of symptoms; inhibiting the disease or symptom, e.g., arresting the progression of the disease or symptom, alleviating the disease or symptom, and promoting remission of the disease or symptom; or terminating the signs of the disease or symptom, and extends to include prophylaxis. "Treatment" also includes achieving a therapeutic benefit and/or a prophylactic benefit. Therapeutic benefit refers to the eradication or improvement of the condition being treated. Moreover, a therapeutic benefit is achieved by the eradication or improvement of one or more physiological symptoms associated with the underlying disease, such that improvement in the patient's disease may be observed even though the patient may still suffer from the underlying disease. Prophylactic benefit means that a patient uses the composition to prevent the risk of a certain disease, or takes the composition upon occurrence of one or more physiological symptoms associated with the disease, even though the disease has not yet been diagnosed.

The term "active ingredient", "therapeutic agent", "active substance" or "active agent" refers to a chemical entity that can effectively treat or prevent a target disorder, disease, or condition. The term "neuropsychiatric disease" refers to the general term for neurological and psychiatric diseases, comprising neurological and/or psychiatric diseases.

With respect to a drug, drug unit or active ingredient, the term "effective amount", "therapeutically effective amount" or "prophylactically effective amount" refers to a sufficient amount of the drug or agent to achieve the desired effect with acceptable side effects. Determination of the effective amount, varying from person to person, depends on the age and general condition of an individual and also depends on a specific active substance. An appropriate effective amount in a particular case can be determined by those skilled in the art according to routine experiments.

As used herein, "individual" includes humans or non-human animals. Exemplary human individuals include those suffering from a disease (e.g., a disease described herein), referred to as patients, or normal individuals. "Non-human animals" in the present invention include all vertebrates, such as non-mammals (e.g., birds, amphibians and reptiles) and mammals, for example, non-human primates, livestock, and/or domesticated animals (e.g., sheep, dogs, cats, cows and pigs).

The term "room temperature" refers to a temperature ranging from 10°C to 40°C. In some embodiments, "room temperature" refers to a temperature ranging from 15°C to 30°C; in some other embodiments, "room temperature" refers to a temperature ranging from 18°C to 25°C.

"Equivalent" or its abbreviation "eq" refers to the equivalent amount of other required materials, based on the stoichiometric relationship of a chemical reaction, wherein the base starting material in each step is used as the reference (1 equivalent).

In the context of the present invention, whether or not the term "approximately" or "about" is used, all values or ranges given shall be deemed to include a tolerance within 10%, suitably within 5%, and particularly within 1%. Alternatively, for a person skilled in the art, the term "approximately" or "about" denotes within an acceptable standard error of the mean value. Whenever a number with a value of N is disclosed, any number falling within the range of N+/-1%, N+/-2%, N+/-3%, N+/-5%, N+/-7%, N+/-8% or N+/-10% shall be explicitly disclosed, wherein "+/-" means plus or minus.

The following detailed description of the present invention is intended to exemplify non-limiting embodiments by way of example, so as to enable those skilled in the art to more fully understand the technical solutions, principles and practical applications of the present invention, so that those skilled in the art may modify and implement the present invention in many forms to best suit the requirements of a particular use.

The compound of the present invention exhibits an inhibitory effect on AAK1, can inhibit cell proliferation, possesses good pharmacokinetic characteristics, high bioavailability, good safety, high selectivity and low toxicity and side effects, and has the advantages of oral administration, rapid absorption, high clearance, etc. Moreover, the compound of the present invention has a good brain penetrability and can be used for preventing and/or treating diabetic neuropathic pain or post-herpetic neuralgia.

### Detailed Description of Embodiments

The present invention is further described below in conjunction with specific examples. It should be understood that these examples are merely used for describing the present invention, rather than limiting the scope of the present invention. In addition, it should be understood that after reading the teachings of the present invention, those skilled in the art can make various changes or modifications to the present invention, and these equivalent forms also fall within the scope defined by the appended claims of the present application.

### Example

Embodiments of the present invention will be described in detail below with reference to examples; however, those skilled in the art will understand that the following examples are only used to illustrate the present invention and should not be regarded as limiting the scope of the present invention. If no specific conditions are indicated in the examples, conventional conditions or the conditions suggested by the manufacturer shall be followed. The reagents or instruments used without indicating the manufacturer are all commercially available conventional products. As used in the present invention, the retention time refers to the peak elution time in analytical liquid chromatography.

### Example 1:

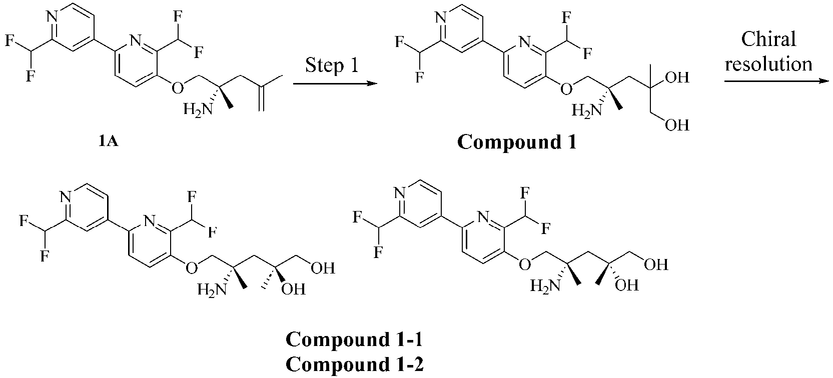

Compound 1A (10.00 g, 25.82 mmol, synthesised using 2-difluoromethyl-4-bromopyridine as a starting material with reference to the synthesis method in patent WO 2023284838 A1) was dissolved in a mixed solution of tetrahydrofuran and water (280 mL, 3 : 1, v : v). The mixture was cooled to 0°C, and then 4-methylmorpholine-4-oxide (9.36 g, 77.46 mmol) was added. The reaction solution was purged with nitrogen, and then an aqueous solution (10 mL) of potassium osmate dihydrate (1.46 g, 3.87 mmol) was added dropwise slowly at 0°C. After the dropwise addition was completed, the reaction solution was reacted at room temperature (28°C) for 16 h. The completion of the reaction was monitored by TLC. The reaction solution was concentrated under reduced pressure to remove most of the tetrahydrofuran, and then diluted in water (100 mL). The mixture was extracted five times with a dichloromethane/methanol system (200 mL, 10 : 1, v : v). The organic phases were combined, dried, and concentrated to afford a crude product. The crude product was separated by silica gel column chromatography (dichloromethane : methanol = 20 : 1 to 5 : 1), followed by reverse-phase separation (acetonitrile : water = 5 : 95 to 95 : 5) to afford 8.00 g of crude compound 1. The sample was further subjected to chiral resolution to afford P1 (retention time: 1.788 min, 785 mg, set as compound **1-1)** and P2 (retention time: 1.987 min, 826 mg, set as compound **1-2).**

Analytical method: instrument: SHIMADZU LC-30AD, column: Chiral OX Column; mobile phase: A: CO₂, B: 0.05% DEA in MEOH; gradient: 5%-40% B in A; flow rate: 3 mL/min; column temperature: 35°C; wavelength: 220 nm.

Preparative method: instrument: Waters 150 Prep-SFC, column: Chiral OX Column; mobile phase: A: CO₂, B: 0.1%NH3•H2O in MEOH; gradient: 35% B gradient elution; flow rate: 100 mL/min; column temperature: 25°C; wavelength: 220 nm; cycle time: 4.2 min; sample preparation: sample concentration 5 mg/ml, methanol solution injection: 1 ml per injection. After separation, the fraction was dried by a rotary evaporator at a bath temperature of 35°C to obtain compound **P1** and compound **P2**.

### Compound 1-1:

¹H NMR (400 MHz, DMSO-*d₆*) δ 8.80 - 8.78 (m, 1H), 8.41 - 8.39 (m, 1H), 8.32 (s, 1H), 8.21 - 8.20 (m, 1H), 7.77 - 7.75 (m, 1H), 7.42 - 6.90 (m, 2H), 4.08 - 4.03 (m, 2H), 3.30 - 3.28 (m, 5H), 3.21 - 3.18 (m, 1H), 1.72 - 1.61 (m, 2H), 1.26 (s, 3H), 1.10 (s, 3H).

LC-MS (ESI): m/z = 418.6 [M+H]⁺.

### Compound 1-2:

¹H NMR (400 MHz, DMSO-*d₆*) δ 8.79 - 8.78 (m, 1H), 8.41 - 8.38 (m, 1H), 8.32 (s, 1H), 8.22 - 8.20 (m, 1H), 7.78 - 7.76 (m, 1H), 7.43 - 6.90 (m, 2H), 4.09 - 4.08 (m, 1H), 3.97 - 3.92 (m, 2H), 3.32 (s, 2H), 3.23 - 3.18 (m, 2H), 3.17 - 3.15 (m, 1H), 1.77 - 1.73 (m, 1H), 1.54 - 1.50 (m, 1H), 1.30 (s, 3H), 1.14 (s, 3H).

LC-MS (ESI): m/z = 418.5 [M+H]⁺.

The synthetic routes for the other disclosed compounds in the present invention are made with reference to the synthetic route of Example 1.

### Biological assay and evaluation

The present invention will be further described and explained below in conjunction with test examples, but these examples are not meant to limit the scope of the present invention.

### Test example 1. In vitro AAK1 enzyme activity assay

Compound stock solution (concentration: 10 mM, dissolved in DMSO) was diluted with DMSO to 0.2 mM and then diluted with DMSO in 5-fold gradient to obtain compound solutions with 10 concentrations. Subsequently, the compound solutions with different concentrations were diluted 50-fold in 1× kinase reaction buffer (containing 40 mM Tris, 20 mM MgCl2, 0.1% BSA and 0.5 mM DTT) for later use. AAK1 (Signalchem, Cat# A01-11G-10) was diluted with 1× kinase reaction buffer to 2-fold the final concentration (final concentrations: 30 nM and 28 nM). AAK1 was added to a 384-well white plate at 2 µL/well, and the compounds were then added at 1 µL/well. The plate was sealed with a plate-sealing film, centrifuged at 1000 rpm for 30 seconds and then placed at room temperature for 10 minutes. A mixed solution of ATP (Promega, Cat# V914B) and substrate Micro2 (GenScript, Cat# PE0890) was formulated at 4-fold the final concentration (for AAK1, the corresponding final concentrations of ATP: 15 µM and 5 µM, and the corresponding final concentration of Micro2: 0.1 mg/mL). To the reaction plate was added the mixed solution of ATP and the substrate at 1 µL/well. The plate was sealed with a plate-sealing film and centrifuged at 1000 rpm for 30 seconds. The reaction was carried out at room temperature for 60 minutes (AAK1). ADP-Glo (Promega, Cat# V9102) was transferred to the 384-well plate at 4 µL/well and centrifugation was carried out at 1000 rpm for 1 minute. The mixture was incubated at 25°C for 40 minutes. A detection solution was transferred to the 384-well plate at 8 µL/well and centrifugation was carried out at 1000 rpm for 1 minute. The resulting mixture was incubated at 25°C for 40 minutes. The RLU (Relative luminescence unit) signal values were read using a Biotek multimode microplate reader, and the percent inhibition was calculated according to the following formula: [1-(LUM compound-LUM positive control) / (LUM negative control-LUM positive control)] × 100. IC₅₀ values were calculated using Graphpad 7.0 software using a four-parameter non-linear fit equation. The specific results are shown in Table 1.

**Table 1 Inhibitory activity against AAK1**

| Compound No. | IC₅₀/nM |
|---|---|
| Compound 1 -1 | ≤ 20 nM |
| Compound 1-2 | ≤ 20 nM |

| | |
|---|---|
| Conclusion: the compounds of the present invention show high inhibitory activity against the AAK1 receptor, for example, the IC50 value of compound 1-2 is 9.62 nM. | |

### Test example 2. hERG potassium channel activity assay

2.1 Experimental preparation: Experimental platform: manual electrophysiological patch-clamp system; Cell line: Chinese hamster ovary (CHO) cell line stably expressing hERG potassium channel.

2.2. Experimental method: In Chinese Hamster Ovary (CHO) cells stably expressing hERG potassium channel, the whole-cell patch-clamp technique was used to record hERG potassium channel current at room temperature. The glass microelectrodes were pulled from glass electrode blanks (BF150-86-10, Sutter) by a glass microelectrode puller. The tip resistance of the microelectrodes after being filled with intrapipette solution was about 2-5 MΩ. The glass microelectrodes were connected to the patch-clamp amplifier by inserting them into an amplifier probe. The clamping voltage and data recording were controlled and recorded by the pClamp 10 software through a computer, with a sampling frequency of 10 kHz and a filtering frequency of 2 kHz. After the whole cell records were obtained, the cells were clamped at -80 mV, and the step voltage that induced the hERG potassium current (I hERG) was depolarised from -80 mV to +20 mV for 2 s, then repolarised to -50 mV, and returned to -80 mV after 1 s. This voltage stimulation was given every 10 s, and the administration process was initiated after the hERG potassium current was confirmed to be stable (at least 1 minute). The compound was administered for at least 1 minute at each test concentration, and at least 2 cells (n ≥ 2) were tested at each concentration.

2.3 Data processing: Data analysis and processing were performed using pClamp 10, GraphPad Prism 5, and Excel software. The degree of inhibition of hERG potassium current (peak value of hERG tail current elicited at -50 mV) at different compound concentrations was calculated according to the following formula: $Inhibition % = \left[1-\left(I/Io\right)\right] \times 100 %$ where Inhibition% represents the percentage of inhibition of hERG potassium current by the compound, and I and Io represent the amplitude of hERG potassium current after and before the administration, respectively.

Compound IC₅₀ was calculated using GraphPad Prism 5 software by fitting according to the following equation: $Y = Bottom + \left(Top-Bottom\right) / \left(1+{10}^{∧}\left(\left(LogIC50-X\right)\times HillSlope\right)\right)$

Among the equation, X represents the Log value of the tested concentration of the test sample, Y represents the inhibition percentage at the corresponding concentration, and Bottom and Top represent the minimum and maximum inhibition percentage, respectively.

**Experimental results:** The IC₅₀ values for inhibitory effects of test compounds on the hERG potassium channel current are shown in the table below:

**Table 2 IC₅₀ values for inhibitory effects of test compounds on hERG potassium channel current**

| Test compound | IC₅₀ (µM) |
|---|---|
| LX9211 | 1.82 |
| Compound 1-1 | 6.73 |
| Compound 1-2 | 13.7 |

| | |
|---|---|
| Note: The structure of LX9211 is disclosed in WO 2015153720 A1 (Example 123). Conclusion: the compounds of the present invention exhibit low cardiotoxicity, and are significantly superior to the control compound. | |

### Test example 3. Pharmacokinetic assay in mice

3.1 Experimental animals: Male ICR mice, 20-25 g, 6 mice/compound, purchased from Chengdu Dossy Experimental Animals Co., Ltd.

3.2 Experimental design: on the day of the experiment, the ICR mice were randomly grouped according to their body weights. The animals were fasted with water available for 12-14 h one day before the administration, and were fed 4 h after the administration.

**Table 3 Administration information**

| Group | Number | Administration information | | | | | |
|---|---|---|---|---|---|---|---|
| | Male | Test compound | Administrat ion dosage (mg/kg) | Administr ation concentra tion (mg/mL) | Administr ation volume (mL/kg) | Collected sample | Administrati on mode |
| G1 | 3 | Compound 1-2 | 2.5 | 0.5 | 5 | Plasma | Intravenous administratio n |
| G2 | 3 | Compound 1-2 | 10 | 1 | 10 | Plasma | Intragastric administratio n |
| G3 | 3 | Compound 1-1 | 10 | 1 | 10 | Plasma | Intragastric administratio n |

### 3.3. Experimental method:

vehicle for intravenous administration: 5%DMA+5%HS-15+90%NS; vehicle for intragastric administration: 0.5% MC; before and after administration, 0.06 mL of blood was taken from the orbit under isoflurane anaesthesia and placed in an EDTAK2 centrifuge tube. The tube was centrifuged at 5000 rpm at 4°C for 10 min, and plasma was collected. The blood sampling time points for both the intravenous administration group and intragastric administration group were 0, 5 min, 15 min, 30 min, 1 h, 2 h, 4 h, 7 h and 24 h. Before analysis and detection, all samples were stored at -80°C, and the samples were quantitatively analysed by LC-MS/MS.

**Table 4 Pharmacokinetic parameters of test compounds in plasma of mice**

| Test compound | Administration mode | CL (mL/min/kg) | Vdₛₛ (L/kg) | AUC₀₋ₜ (hr*ng/mL) | T_{1/2} (h) |
|---|---|---|---|---|---|
| Compound 1-2 | Intravenous administration (2.5 mg/kg) | 57.8 | 10.5 | 630 | 2.53 |
| Compound 1-2 | Intragastric administration (10 mg/kg) | - | - | 3525 | 3.09 |
| Compound 1-1 | Intragastric administration (10 mg/kg) | - | - | 4400 | 4.82 |

| | | | | | |
|---|---|---|---|---|---|
| Vehicle for administration: 0.5% MC Conclusion: the compounds of the present invention, such as the example compounds, have good pharmacokinetic characteristics in mice. | | | | | |

### Test example 4. Pharmacokinetic assay in rats

4.1 Experimental animals: Male SD rats, about 220 g, 6-8 weeks old, 6 rats/compound, purchased from Chengdu Dossy Experimental Animals Co., Ltd.

4.2 Experimental design: on the day of the experiment, 6 SD rats were randomly grouped according to their body weights. The animals were fasted with water available for 12-14 h one day before the administration, and were fed 4 h after the administration.

**Table 5 Administration information**

| Group | Num ber | Administration information | | | | | |
|---|---|---|---|---|---|---|---|
| | Male | Test compound | Admini stration dosage (mg/kg) | Administration concentration (mg/mL) | Administ ration volume (mL/kg) | Collected sample | Administrat ion mode |
| G1 | 3 | Compound | 2.5 | 0.5 | 5 | Plasma | Intravenous administrati on |
| G2 | 3 | Compound | 5 | 1 | 5 | Plasma | Intragastric administrati on |

| | | | | | | | |
|---|---|---|---|---|---|---|---|
| Vehicle for administration: 0.5% MC | | | | | | | |

Before and after the administration, 0.1 ml of blood was taken from the orbit under isoflurane anaesthesia and placed in an EDTAK2 centrifuge tube. The tube was centrifuged at 5000 rpm at 4°C for 10 min, and plasma was collected. Blood sampling time points for the intravenous administration group: 0, 5 min, 15 min, 30 min, 1 h, 2 h, 4 h, 6 h, 8 h and 24 h; blood sampling time points for the intragastric administration group: 0, 5 min, 15 min, 30 min, 1 h, 2 h, 4 h, 6 h, 8 h and 24 h. Before analysis and detection, all samples were stored at -80°C.

The experimental results are as shown in Table 6.

**Table 6 Pharmacokinetic parameters of test compounds in plasma of rats**

| Test compound | Administration mode | CL (mL/min/kg) | AUC₀₋ₜ (hr*ng/mL) | T_{1/2} (h) |
|---|---|---|---|---|
| Compound 1-2 | Intragastric administration (5 mg/kg) | 53.7 | 4047 | 4.52 |

| | | | | |
|---|---|---|---|---|
| Conclusion: the compounds of the present invention, such as compound 1-2, have good pharmacokinetic characteristics in rats. | | | | |

### Test example 5. Liver microsomal stability assay

In this experiment, liver microsomes of five species, including human, monkey, dog, rat and mouse, were used as in vitro models to evaluate the metabolic stability of the test compound.

At 37°C, 1 µM of the test compound was co-incubated with microsomal protein and coenzyme NADPH. At given time points of the reaction (5 min, 10 min, 20 min, 30 min, and 60 min), the reaction was terminated by adding ice-cold acetonitrile containing an internal standard. The LC-MS/MS method was used to measure the concentration of the test compound in the sample. T_{1/2} was calculated using the ln value of the percentage of drug remaining in the incubation system and the incubation time. In addition, the intrinsic clearance in liver microsomes CL_{int(mic)} and the intrinsic clearance in liver CL_{int(Liver)} were further calculated.

**Table 7 Results of test compounds in rat liver microsomal model**

| Test compound | **Percentage of drug remaining after 60 min of incubation** |
|---|---|
| Compound 1-1 | 93.7% |
| Compound 1-2 | > 99% |

| | |
|---|---|
| Conclusion: the compounds of the present invention, such as the example compounds, are metabolically stable in liver microsomes across five species. | |

## Claims

1. A compound represented by general formula (I), or a stereoisomer or a pharmaceutically acceptable salt thereof: 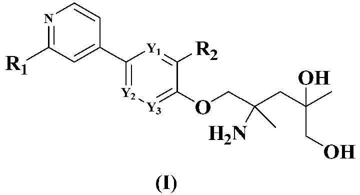 wherein:
Y₁, Y₂ and Y₃ are each independently CRₐ or N;
Rₐ is hydrogen, deuterium, halogen, hydroxyl, cyano, amino, C₁₋₆ alkyl, C₁₋₆ deuteroalkyl, C₁₋₆ haloalkyl, C₁₋₆ alkoxy, C₁₋₆ alkylthio, C₁₋₆ alkylamino, C₃₋₈ cycloalkyl, 3- to 8-membered heterocycloalkyl containing 1-3 heteroatoms selected from N, O or S, C₆₋₁₀ aryl, or 5- to 10-membered heteroaryl containing 1-3 heteroatoms selected from N, O or S;
R₁ and R₂ are each independently hydrogen, deuterium, halogen, cyano, amino, mercapto, carbamoyl, C₁₋₆ alkyl, C₁₋₆ deuteroalkyl, C₁₋₆ haloalkyl, C₁₋₆ alkoxy, C₁₋₆ alkylthio, C₁₋₆ alkylamino, C₁₋₆ hydroxyalkyl, halogenated C₁₋₆ alkoxy, deuterated C₁₋₆ alkoxy, C₃₋₈ cycloalkyl, 3- to 8-membered heterocycloalkyl containing 1-3 heteroatoms selected from N, O or S, C₆₋₁₀ aryl, 5- to 10-membered heteroaryl containing 1-3 heteroatoms selected from N, O or S, -C(O)C₁₋₆ alkyl, - NHC(O)C₁₋₆ alkyl, -NHC(O)OC₁₋₆ alkyl, -NHC(O)NHC₁₋₆ alkyl, -NHC(O)C₃₋₈ cycloalkyl, -NHC(O)OC₃₋₈ cycloalkyl, -NHC(O)C₄₋₆ heterocycloalkyl or - NHC(O)OC₄₋₆ heterocycloalkyl;
and the following compounds are excluded:

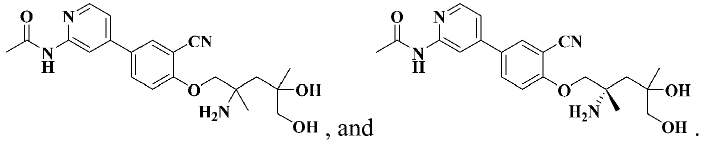

2. The compound represented by general formula (I) or the stereoisomer or pharmaceutically acceptable salt thereof according to claim 1, **characterized in that** one or more of the following conditions are satisfied:
(1) Y₁, Y₂ and Y₃ are each independently CH or N; preferably Y₁, Y₂ and Y₃ are each independently CH or N, and Y₁, Y₂ and Y₃ are not CH simultaneously; more preferably Y₁ is N, Y₂ and Y₃ are CH, or Y₂ is N, Y₁ and Y₃ are CH, or Y₃ is N, Y₁ and Y₂ are CH;
(2) Rₐ is hydrogen, halogen or C₁₋₆ alkyl, preferably hydrogen, halogen or C₁₋₃ alkyl, more preferably hydrogen;
(3) R₁ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, -NHC(O)OC₁₋₆ alkyl or -NHC(O)NHC₁₋₆ alkyl, preferably C₁₋₃ haloalkyl or -NHC(O)OC₁₋₃ alkyl, more preferably -CHF₂ or -NHC(O)OCH₃;
(4) R₂ is hydrogen, deuterium, halogen, cyano, C₁₋₆ alkyl, C₁₋₆ deuteroalkyl, C₁₋₆ haloalkyl or C₁₋₆ alkoxy, preferably cyano, C₁₋₃ alkyl or C₁₋₃ haloalkyl, more preferably cyano, methyl or -CHF₂;
(5) the compound represented by general formula (I) is selected from: 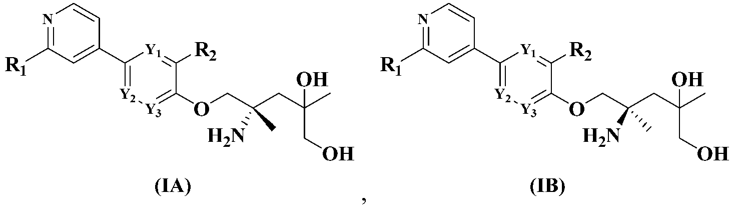 or a mixture thereof.

3. The compound represented by general formula (I) or the stereoisomer or pharmaceutically acceptable salt thereof according to claim 1, **characterized in that** the general formula (I) is further as represented by general formula (II): 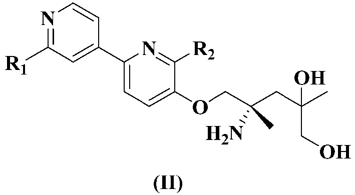 wherein:
R₁ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, -NHC(O)OC₁₋₆ alkyl or -NHC(O)NHC₁₋₆ alkyl;
R₂ is hydrogen, deuterium, halogen, cyano, C₁₋₆ alkyl, C₁₋₆ deuteroalkyl, C₁₋₆ haloalkyl, C₁₋₆ alkoxy, -C(O)C₁₋₆ alkyl, -NHC(O)C₁₋₆ alkyl, -NHC(O)OC₁₋₆ alkyl or -NHC(O)NHC₁₋₆ alkyl.

4. The compound represented by general formula (I) or the stereoisomer or pharmaceutically acceptable salt thereof according to claim 3, **characterized in that** the general formula (II) is further as represented by general formula (IIA) or general formula (IIB): 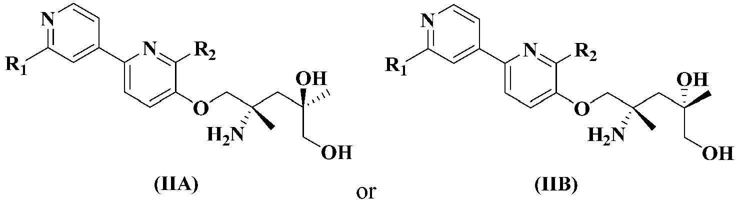 wherein
R₁ and R₂ are as defined in claim 3.

5. The compound represented by general formula (I) or the stereoisomer or pharmaceutically acceptable salt thereof according to any one of claims 1-4, **characterized in that** the compound is selected from the following compounds:

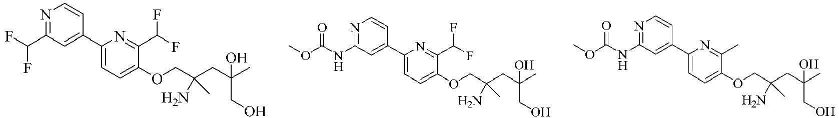

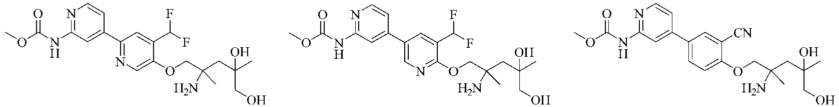

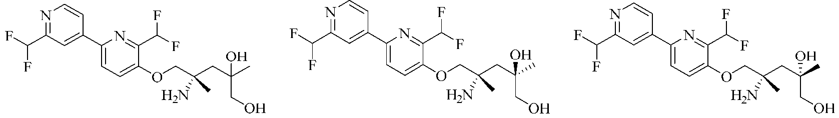

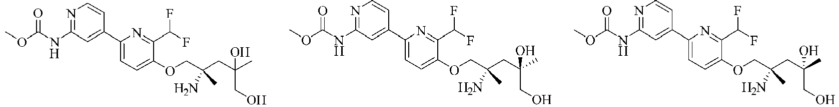

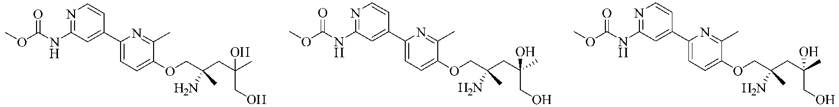

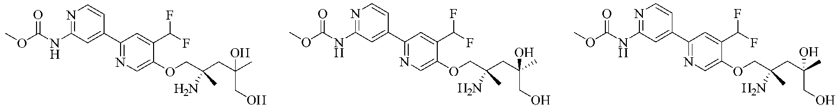

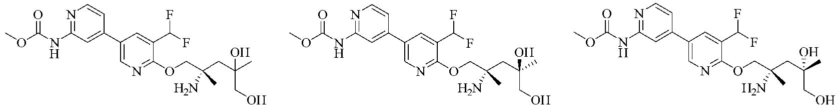

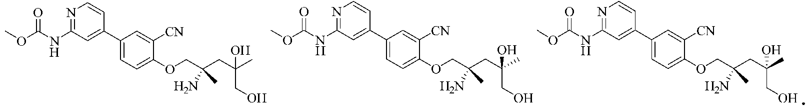

6. A pharmaceutical composition, comprising a therapeutically effective dose of the compound, or the stereoisomer or pharmaceutically acceptable salt thereof according to any one of claims 1-5, and one or more pharmaceutically acceptable carriers or excipients.

7. The pharmaceutical composition according to claim 6, comprising 1-1500 mg of the compound, or the stereoisomer or pharmaceutically acceptable salt thereof according to any one of claims 1-5, and one or more pharmaceutically acceptable carriers or excipients.

8. Use of the compound, or the stereoisomer or pharmaceutically acceptable salt thereof according to any one of claims 1-5, or the pharmaceutical composition according to claim 6 or 7 in the preparation of a medicament, preferably the medicament is a medicament for preventing and/or treating AAK1-mediated diseases.

9. The use according to claim 8, wherein the AAK1-mediated disease is diabetic neuropathic pain or post-herpetic neuralgia.

10. A method for treating a disease in a mammal, **characterized in that** the method comprises administering to a subject a therapeutically effective amount of the compound, or the stereoisomer or pharmaceutically acceptable salt thereof according to any one of claims 1-5, or the pharmaceutical composition according to claim 6 or 7, wherein the therapeutically effective amount is preferably 1-1500 mg, and the disease is preferably diabetic neuropathic pain or post-herpetic neuralgia.
